# EUROPEAN PATENT APPLICATION

(11) **EP 3 890 447 A1**
(43) Date of publication of application: **06.10.2021**
(21) Application number: 19890252.0
(22) Date of filing: 26.11.2019
(51) Int. Cl.: H05B 47/115, A61L 2/10, H01L 33/00

(54) **ILLUMINATION DEVICE**

(30) Priority: 27.11.2018 JP 2018221357
(71) Applicant: Kyocera Corporation, Kyoto-shi, Kyoto 612-8501 (JP)
(72) Inventor: KATOU,Hidetaka, Kyoto-shi, Kyoto 612-8501 (JP); KUSANO,Tamio, Kyoto-shi, Kyoto 612-8501 (JP); NIZUKA,Takeshi, Kyoto-shi, Kyoto 612-8501 (JP); ANDO,Yoshiki, Kyoto-shi, Kyoto 612-8501 (JP)
(74) Representative: Viering, Jentschura & Partner mbB Patent- und Rechtsanwälte
(86) International application number: PCT/JP2019/046133
(87) International publication number: WO 2020/111051

(57) **Abstract**

An illumination device 10 according to an embodiment of the present disclosure includes a light emitting device, a control unit, and a sensor. The light emitting device 1 emits light having a first peak wavelength λ1 in a range of 360 to 430 nm and a second peak wavelength λ2 in a range of 430 to 700 nm. The control unit 7 controls an emission spectrum of the light emitted from the light emitting device 1. The sensor 8 includes a sensor unit 81 and transmits a signal received by the sensor unit 81 to the control unit 7. The control unit 7 controls the emission spectrum based on a signal detected by the sensor 8.

## Description

### Technical Field

The present disclosure relates to an illumination device.

### Background Art

In recent years, illumination devices using semiconductor light emitting elements such as LEDs (Light Emitting Diodes) as light sources are used instead of fluorescent lamps and light bulbs. For example, an illumination device using a light emitting element as a light source is also used as a light source for appearance inspection of a coated surface of a household appliance, a passenger vehicle, or the like.

The semiconductor light emitting element has a narrow wavelength band of radiation light, and is capable of emitting only light of one color. When it is desired to use white light as illumination light, a plurality of semiconductor light emitting elements having different wavelength bands of radiation light is prepared, and white light is provided by mixing colors of a plurality of beams of radiation light. Alternatively, a plurality of phosphors that emits fluorescence in different wavelength bands with excitation light of the same wavelength is prepared, and white light is provided by radiation light from a semiconductor light emitting element and through mixture of colors of a plurality of beams of fluorescence that is excited with the radiation light from the semiconductor light emitting element and that emits light. By using such a method of mixing colors, it is possible to produce a light source having a spectrum corresponding to a purpose other than white light (see Japanese Unexamined Patent Application Publication No. 2015-126160).

However, the technology disclosed in Japanese Unexamined Patent Application Publication No. 2015-126160 neither describes nor expects control of the emission intensity and the emission spectrum of the illumination device.

### Summary of Invention

An illumination device according to an embodiment of the present disclosure includes a light emitting device, a control unit, and a sensor. The light emitting device emits light having a first peak wavelength in a range of 360 to 430 nm and a second peak wavelength in a range of 430 to 700 nm. The control unit controls an emission spectrum of the light emitted from the light emitting device. The sensor includes a sensor unit and transmits a signal received by the sensor unit to the control unit. The control unit controls the emission spectrum based on a signal detected by the sensor.

An illumination device according to an embodiment of the present disclosure includes a plurality of light emitting devices, a control unit, and a sensor. The plurality of light emitting devices emits light having a first peak wavelength in a range of 360 to 430 nm and a second peak wavelength in a range of 430 to 700 nm. The control unit controls an emission spectrum of the light emitted from the light emitting devices. The sensor includes a sensor unit and transmits a signal received by the sensor unit to the control unit. The control unit controls the emission spectrum based on a signal detected by the sensor.

An illumination device according to an embodiment of the present disclosure includes a first light emitting device, a second light emitting device, a control unit, and a sensor. The first light emitting device emits light having a first peak wavelength in a range of 360 to 430 nm. The second light emitting device emits light having a second peak wavelength in a range of 430 to 700 nm. The control unit controls an emission spectrum obtained by combining a first emission spectrum of the light emitted from the first light emitting device and a second emission spectrum of the light emitted from the second light emitting device. The sensor includes a sensor unit and transmits a signal received by the sensor unit to the control unit. The control unit controls the emission spectrum based on a signal detected by the sensor.

### Brief Description of Drawings

[Fig. 1] Fig. 1 is an external perspective view of a light emitting device according to an embodiment of the present disclosure.
[Fig. 2] Fig. 2 is a cross-sectional view when the light emitting device illustrated in Fig. 1 is cut along a plane indicated by an imaginary line.
[Fig. 3] Fig. 3 is a cross-sectional view when another embodiment of the light emitting device illustrated in Fig. 1 is cut along the plane indicated by the imaginary line.
[Fig. 4] Fig. 4 is an enlarged view of the light emitting device illustrated in Fig. 2.
[Fig. 5] Fig. 5 is a graph presenting a spectrum of external radiation light of the light emitting device according to the embodiment of the present disclosure.
[Fig. 6] Fig. 6 is a graph presenting a spectrum of external radiation light of the light emitting device according to the embodiment of the present disclosure.
[Fig. 7] Fig. 7 is an external perspective view of an illumination device including the light emitting device according to the embodiment of the present disclosure.
[Fig. 8] Fig. 8 is an exploded perspective view of the illumination device according to the embodiment of the present disclosure.
[Fig. 9] Fig. 9 is a perspective view illustrating a state in which a translucent substrate is removed from a housing of the illumination device according to the embodiment of the present disclosure.
[Fig. 10] Fig. 10 is a graph presenting a sterilization effect when the light emitting device or the illumination device according to the embodiment of the present disclosure is used.
[Fig. 11] Fig. 11 is a diagram illustrating a configuration of the illumination device according to the embodiment of the present disclosure.
[Fig. 12] Fig. 12 is a diagram illustrating a configuration of the illumination device according to the embodiment of the present disclosure.
[Fig. 13] Fig. 13 is a configuration diagram of the illumination device according to the embodiment of the present disclosure.
[Fig. 14] Fig. 14 is a cross-sectional view illustrating a configuration of an illumination device according to another embodiment of the present disclosure.
[Fig. 15] Fig. 15 is a graph presenting a spectrum of external radiation light of the illumination device according to the other embodiment of the present disclosure.

### Description of Embodiments

Hereinafter, a light emitting device and an illumination device according to embodiments of the present disclosure will be described with reference to the drawings.

### <Configurations of Light Emitting Device and Illumination Device>

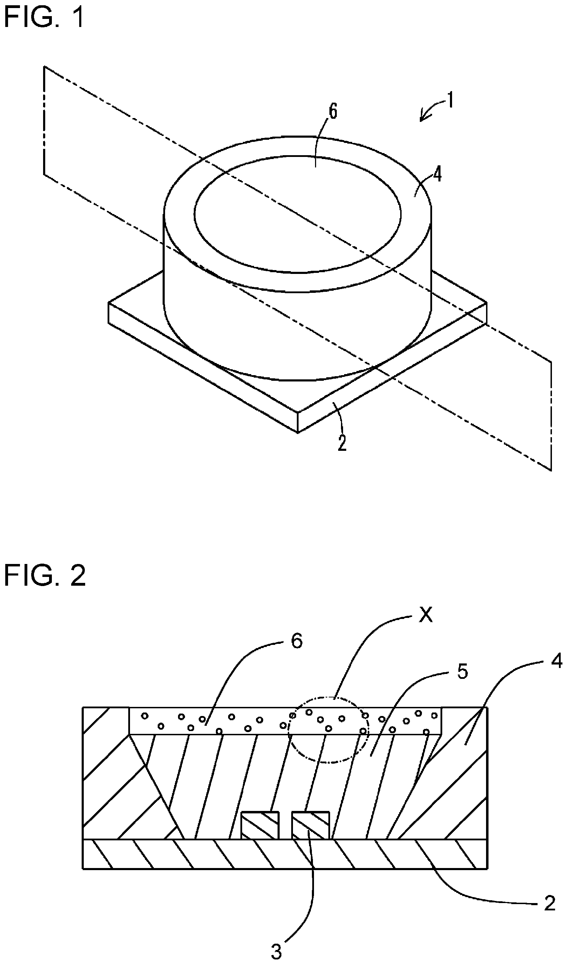

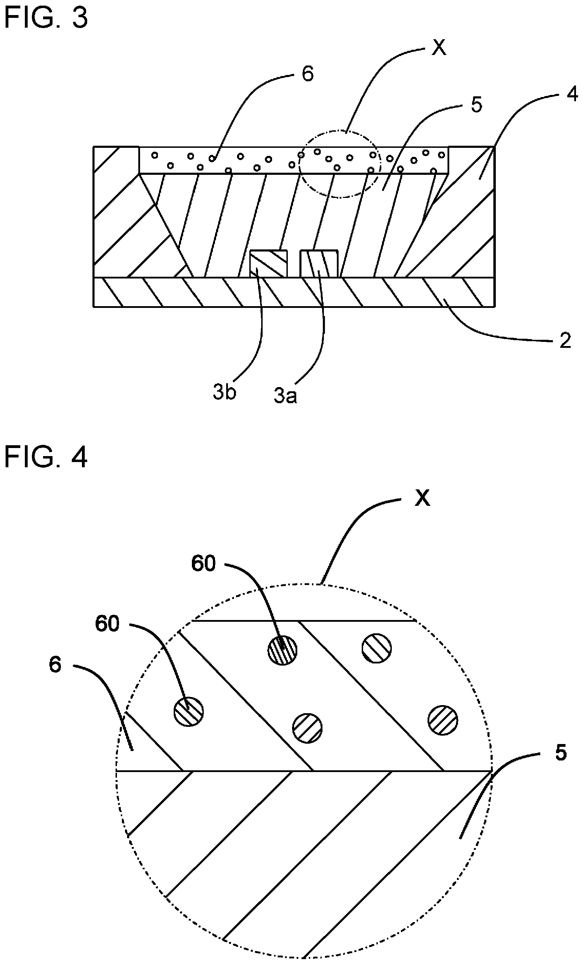

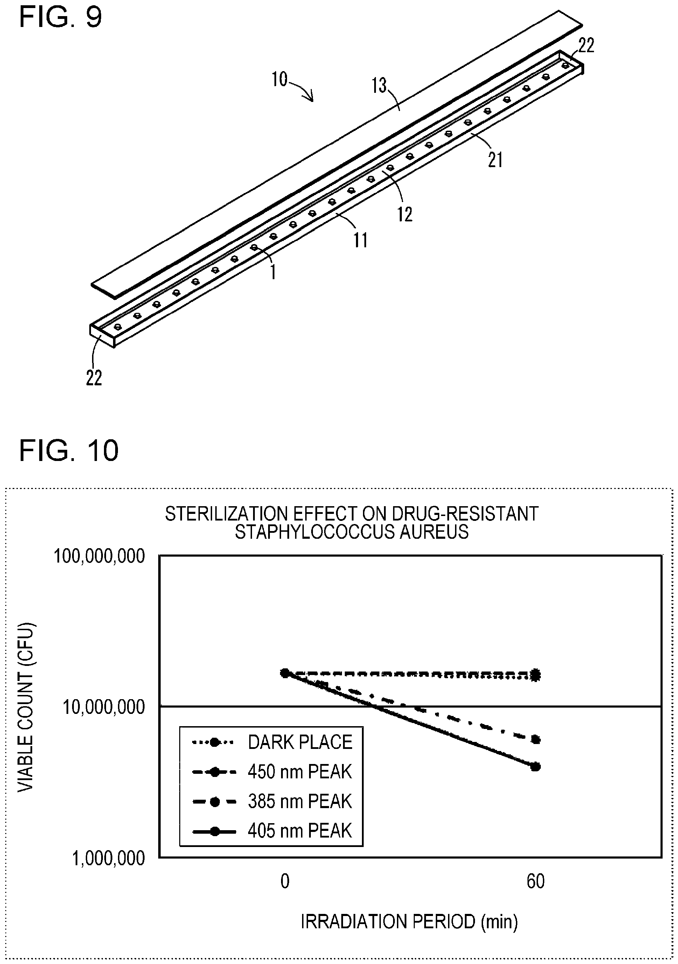

Fig. 1 is an external perspective view of a light emitting device according to an embodiment of the present disclosure. Fig. 2 is a cross-sectional view when the light emitting device illustrated in Fig. 1 is cut along a plane indicated by an imaginary line. Fig. 3 is a cross-sectional view when the light emitting device illustrated in Fig. 1 is cut along the plane indicated by the imaginary line. Fig. 10 is a graph presenting a sterilization effect when the light emitting device or the illumination device according to the embodiment of the present disclosure is used. In these drawings, a light emitting device 1 includes a substrate 2, a light emitting element 3, a frame body 4, a sealing member 5, and a wavelength conversion member 6.

The light emitting element 3 is located on the substrate 2. The frame body 4 is located on the substrate 2 to surround the light emitting element 3. The inner space surrounded by the frame body 4 is filled with the sealing member 5 while part of an upper portion of the space surrounded by the frame body 4 is left unfilled. The wavelength conversion member 6 is housed in the frame body 4 along the upper surface of the sealing member 5, in part of the upper portion of the inner space surrounded by the frame body 4. The light emitting element 3 is, for example, an LED (Light Emitting Diode) or ?? (Laser Diode), and emits light toward the outside when electrons and holes in a pn junction using a semiconductor are recombined.

The substrate 2 is a substrate mainly made of an insulating material. The insulating material is, for example, a ceramic material such as alumina or mullite; or a glass ceramic material. Alternatively, the substrate 2 is made of a composite-based material obtained by mixing a plurality of these materials. As the substrate 2, a polymer resin in which metal oxide particles capable of adjusting thermal expansion of the substrate 2 are dispersed can be used.

A wiring conductor that electrically connects the inside and outside of the substrate 2 is provided at least on the main surface of the substrate 2 or inside the substrate 2. The wiring conductor is made of, for example, a conductive material such as tungsten, molybdenum, manganese, or copper. When the substrate 2 is made of a ceramic material, for example, a metal paste obtained by adding an organic solvent to powder of tungsten or the like is printed in a predetermined pattern on a ceramic green sheet that is to be the substrate 2. Then, plural ceramic green sheets are stacked and fired to obtain the substrate 2. For example, a plating layer of nickel, gold, or the like is formed on a surface of the wiring conductor to inhibit oxidation. A metal reflection layer may be located on the upper surface of the substrate 2 with a gap from the wiring conductor and the plating layer in order to efficiently reflect light upward of the substrate 2. The metal reflection layer is, for example, aluminum, silver, gold, copper, or platinum.

The light emitting element 3 is mounted on the main surface of the substrate 2. The light emitting element 3 is electrically connected onto the plating layer deposited on the surface of the wiring conductor formed on the main surface of the substrate 2 via, for example, a brazing material or solder. The light emitting element 3 includes a translucent base and an optical semiconductor layer formed on the translucent base. The translucent base may be any object on which an optical semiconductor layer can be grown by a chemical vapor deposition method such as a metal-organic chemical vapor deposition method or a molecular beam epitaxial deposition method. Examples of the material used for the translucent base include sapphire, gallium nitride, aluminum nitride, zinc oxide, zinc selenide, silicon carbide, silicon, and zirconium diboride. The thickness of the translucent base is, for example, 50 µm or more and 1000 µm or less.

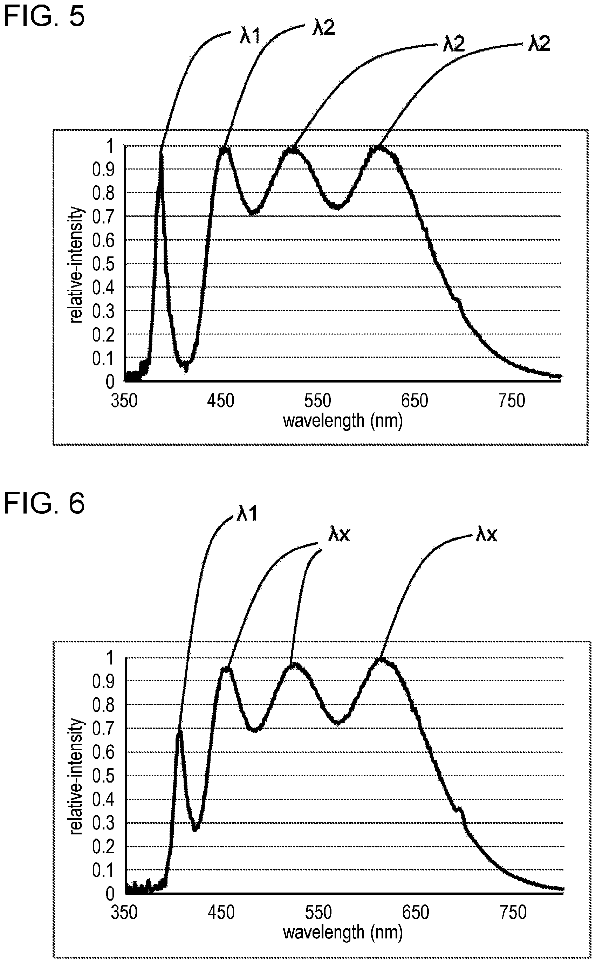

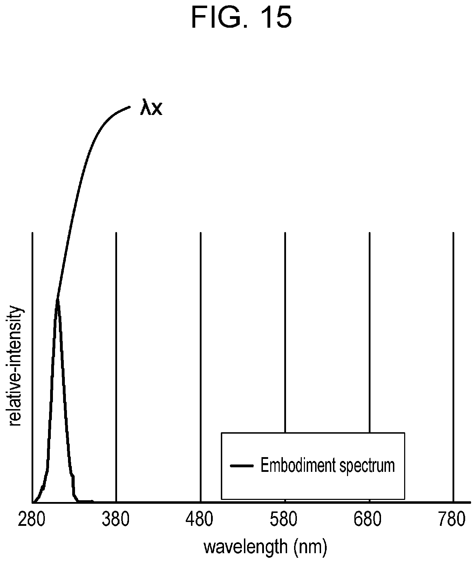

The optical semiconductor layer includes a first semiconductor layer formed on the translucent base, a light emitting layer formed on the first semiconductor layer, and a second semiconductor layer formed on the light emitting layer. The first semiconductor layer, the light emitting layer, and the second semiconductor layer can be made of, for example, a Group III nitride semiconductor, a Group III-V semiconductor such as gallium phosphide or gallium arsenide, or a Group III nitride semiconductor such as gallium nitride, aluminum nitride, or indium nitride. The thickness of the first semiconductor layer is, for example, 1 µm or more and 5 µm or less, the thickness of the light emitting layer is, for example, 25 nm or more and 150 nm or less, and the thickness of the second semiconductor layer is, for example, 50 nm or more and 600 nm or less. As illustrated in Figs. 5, 6, and 15, the light emitting element 3 configured as described above can emit excitation light in a wavelength range of, for example, 280 nm or more and 450 nm or less.

The frame body 4 is made of, for example, a ceramic material such as aluminum oxide, titanium oxide, zirconium oxide, or yttrium oxide; a porous material; or a resin material mixed with powder made of a metal oxide such as aluminum oxide, titanium oxide, zirconium oxide, or yttrium oxide. The frame body 4 is connected to the main surface of the substrate 2 via, for example, resin, a brazing material, or solder. The frame body 4 is provided on the main surface of the substrate 2 to surround the light emitting element 3 with a gap from the light emitting element 3. The frame body 4 has an inclined inner wall surface that is formed to expand outward as the distance from the main surface of the substrate 2 increases. The inner wall surface of the frame body 4 functions as a reflection surface for the excitation light emitted from the light emitting element 3. When the inner wall surface of the frame body 4 has a circular shape in plan view, light emitted by the light emitting element 3 can be uniformly reflected outward by the reflection surface.

For the inclined inner wall surface of the frame body 4, for example, a metal layer made of tungsten, molybdenum, manganese, or the like and a plating layer made of nickel, gold, or the like covering the metal layer may be formed on the inner peripheral surface of the frame body 4 made of a sintered material. This plating layer has a function of reflecting light emitted by the light emitting element 3. The inclination angle of the inner wall surface of the frame body 4 is set to an angle of, for example, 55 degrees or more and 70 degrees or less with respect to the main surface of the substrate 2.

The inner space surrounded by the substrate 2 and the frame body 4 is filled with the light-transmitting sealing member 5. The sealing member 5 seals the light emitting element 3 and allows the light emitted from the inside of the light emitting element 3 to be extracted to the outside. The sealing member 5 also has a function of transmitting light emitted by the light emitting element 3 and extracted to the outside of the light emitting element 3. The inner space surrounded by the substrate 2 and the frame body 4 is filled with the sealing member 5 while part of the space surrounded by the frame body 4 is left unfilled. For example, a translucent insulating resin such as a silicone resin, an acrylic resin, or an epoxy resin; or a translucent glass material is used for the sealing member 5. The refractive index of the sealing member 5 is set to, for example, 1.4 or more and 1.6 or less.

The wavelength conversion member 6 is located along the upper surface of the sealing member 5 in an upper portion of the inner space surrounded by the substrate 2 and the frame body 4. The wavelength conversion member 6 is formed so as to be housed in the frame body 4. The wavelength conversion member 6 has a function of converting the wavelength of the light emitted by the light emitting element 3. That is, the light emitted from the light emitting element 3 enters the inside of the wavelength conversion member 6 through the sealing member 5. At this time, phosphors contained in the wavelength conversion member 6 are excited by the light emitted from the light emitting element 3, and emit fluorescence from the phosphors. In addition, the wavelength conversion member 6 transmits part of the light from the light emitting element 3 and emits the light. The wavelength conversion member 6 is made of, for example, a translucent insulating resin such as a fluorocarbon resin, a silicone resin, an acrylic resin, or an epoxy resin; or a translucent glass material. The phosphors are contained in the insulating resin or the glass material. The phosphors are uniformly dispersed in the wavelength conversion member 6. The phosphors contained in the light emitting element 3 and the wavelength conversion member 6 are selected such that the emission spectrum of the light emitted from the light emitting device 1 is an emission spectrum as illustrated in Fig. 4.

As illustrated in Figs. 5 and 6, in the light emitting device 1 according to the embodiment of the present disclosure, the light emitting element 3 is used, where a first peak wavelength λ1 is present in a range of 360 to 430 nm. As the phosphors, for example, a phosphor that emits blue fluorescence and a phosphor that emits blue-green fluorescence are used, where a second peak wavelength λ2 is present in a range of 430 to 700 nm. In addition, a phosphor that emits green fluorescence, a phosphor that emits red fluorescence, and a phosphor that emits fluorescence in the near infrared region may be further used.

As each phosphor, examples of the phosphor exhibiting blue color include BaMgAl₁₀O₁₇:Eu, (Sr,Ca,Ba)₁₀(PO₄)₆Cl₂:Eu, and (Sr,Ba)₁₀(PO₄)₆Cl₂:Eu, and examples of the phosphor exhibiting blue-green color include (Sr,Ba,Ca)₅(PO₄)₃Cl:Eu and Sr₄Al₁₄O₂₅:Eu. Examples of the phosphor exhibiting green color include SrSi₂(O,Cl)₂N₂:Eu, (Sr,Ba,Mg)₂SiO₄:Eu²⁺, ZnS:Cu, Al, and Zn₂SiO₄:Mn. Examples of the phosphor exhibiting red color include Y₂O₂S:Eu, Y₂O₃:Eu, SrCaClAlSiN₃:Eu²⁺, CaAlSiN₃:Eu, and CaAlSi(ON)₃:Eu. Examples of the phosphor exhibiting the near infrared region include 3Ga₅O₁₂:Cr.

When a plurality of light emitting devices 1 is provided, a first light emitting device 1a may have a first peak wavelength λ1 in a range of 360 to 430 nm, and a second light emitting device 1b may have a second peak wavelength λ2 in a range of 430 to 700 nm. In this case, the second light emitting device 1b may be a blue light emitting diode and the phosphors may be those using yellow.

### <Configuration of Illumination Device>

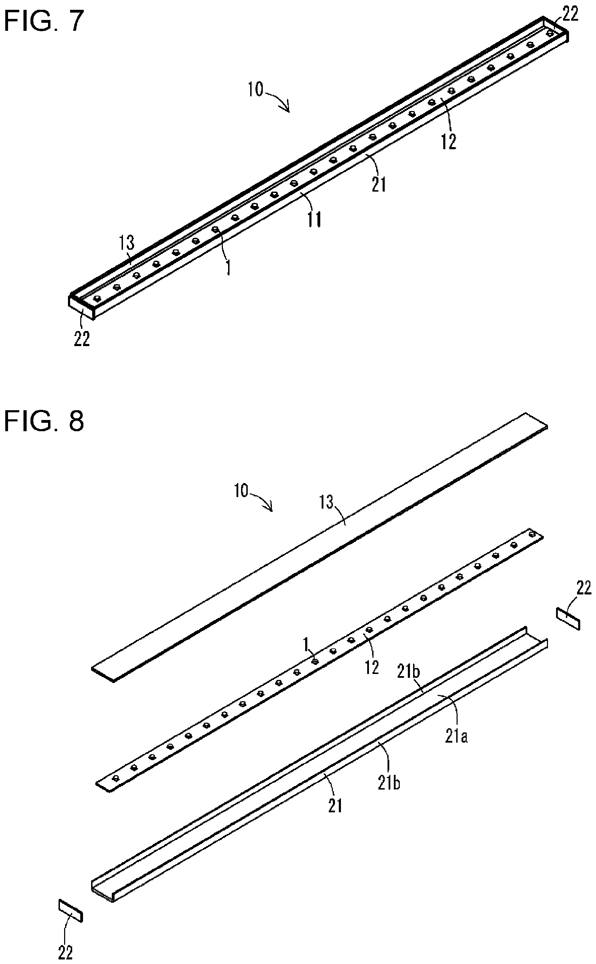

As illustrated in Figs. 7 to 9, and Figs. 11 and 12, an illumination device 10 according to the embodiment of the present disclosure includes the light emitting device 1 described above, a control unit 7, and a sensor 8. Hereinafter, description is provided with reference to the drawings. While the spectrum of the emitted light in the illumination device 10 is referred to as an emission spectrum, the emission spectrum of the light emitted from the light emitting device 1 can be identified as an emission spectrum A, the emission spectrum of the light emitted from the plurality of light emitting devices 1 can be identified as an emission spectrum B, and the emission spectrum of the light emitted from the first light emitting device 1a and the second light emitting device 1b can be identified as an emission spectrum C.

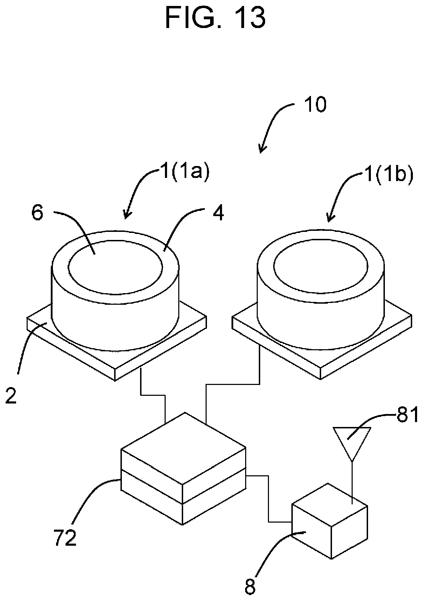

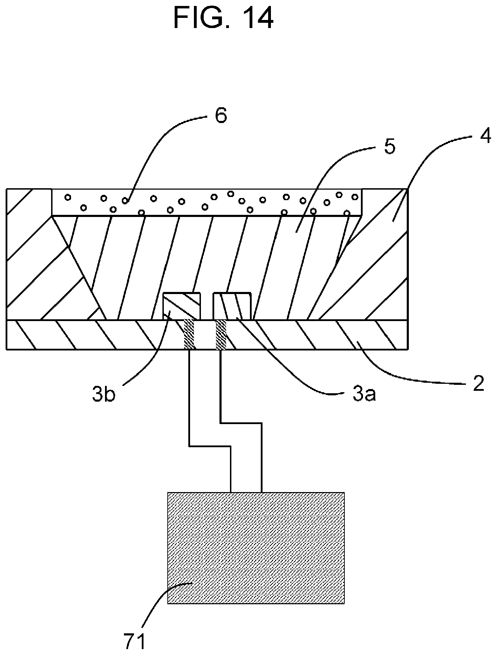

The control unit 7 is a part that controls the emission spectrum of the light emitted from the light emitting device 1. For example, the control unit 7 can adjust emission intensity of the light emitted from the light emitting device 1. When the light emitting device 1 includes a plurality of light emitting elements, as illustrated in Fig. 14, the control unit 7 can adjust to which light emitting element a voltage is applied, how much voltage or current is applied, and the like, with respect to the circuit of the light emitting device 1. At this time, which light emitting element is selected is adjusted by a first selection unit 71. When the plurality of light emitting devices 1 is provided or when the light emitting device 1 (the light emitting device 1a) and the second light emitting device 1b having the second peak wavelength λ2 are provided, as illustrated in Fig. 13, it is also possible to adjust which light emitting device is to emit light, which light emitting device has a higher emission intensity, and the like. That is, the emission spectrum of the illumination device 10 is a combination of a first emission spectrum that is the spectrum of the light emitted from the first light emitting device 1a, and a second emission spectrum that is the spectrum of the light emitted from the second light emitting device 1b. The intensity of the first emission spectrum can be adjusted by adjusting the voltage or current applied to the first light emitting device 1a, and the intensity of the second emission spectrum can be adjusted by adjusting the voltage or current applied to the second light emitting device 1b. In this way, the emission spectrum of the illumination device 10 can be controlled. At this time, which light emitting device is selected is adjusted by a second selection unit 72. The control unit 7 may control the light emitting device 1 described above based on a signal or information received from the outside through wireless communication or the like. The control unit 7 may include an arithmetic device such as a CPU, a memory, and the like.

The sensor 8 includes a sensor unit 81 and is a part that transmits a signal received by the sensor unit 81 to the control unit 7. That is, the sensor 8 detects a signal. Specifically, the sensor unit 81 receives a signal and transmits the signal to the control unit 7. The sensor unit 81 is a semiconductor device or the like. The sensor 8 is, for example, a particle sensor, an odor sensor, a bacteria detection sensor, a color identification sensor, or an optical sensor such as a distance measurement sensor or an infrared sensor. The sensor 8 may be coupled to the main body of the illumination device 10 by wire, or may be independent of the main body of the illumination device 10 and may transmit a signal or information through wireless communication. When the sensor 8 transmits a signal through wireless communication, it is possible to control the emission spectrum based on information from a location farther than the illumination device. Alternatively, a plurality of sensors 8 may be connected to the main body of the illumination device.

In the illumination device 10, the control unit 7 controls the emission spectrum emitted from the light emitting device 1 based on a signal detected by the sensor 8. Specifically, in the case where the sensor 8 is a particle sensor, when the sensor 8 detects specific particles, the control unit 7 performs control such as increasing the output to increase the emission intensity. In the case where the first light emitting device 1a and the second light emitting device 2b are provided, when the sensor 8 detects particles, the control unit 7 may perform control to increase the emission intensity of the first light emitting device 1a. In the case where the plurality of light emitting devices 1 is provided, the emission intensity of all the light emitting devices 1 may be increased when the sensor 8 detects particles.

Likewise, in the case where the sensor 8 is an odor sensor, when the sensor 8 detects a specific odor, the control unit 7 performs control such as increasing the output to increase the emission intensity. In the case where the sensor 8 is a bacteria detection sensor, when the sensor 8 detects specific bacteria, the control unit 7 performs control such as increasing the output to increase the emission intensity. In the case where the sensor 8 is an optical sensor such as an infrared sensor, when the sensor 8 detects a human or the like, the control unit 7 performs control such as decreasing the output to decrease the emission intensity. Specifically, in the case where the first light emitting device 1a and the second light emitting device 2b are provided, when the sensor 8 detects bacteria, the control unit 7 may perform control such as increasing the emission intensity of the first light emitting device 1a with respect to the skin or eyes of a human body. In the case where the plurality of light emitting devices 1 is provided, the emission intensity of all the light emitting devices 1 may be increased when the sensor 8 detects bacteria.

The illumination device 10 may have a sterilization effect by having the first peak wavelength λ1. At this time, by inputting information on germs to be sterilized to the control unit 7, when the sensor 8 detects the germs, it is possible to increase the output of the light emitting device having the light of the first peak wavelength. The wavelength of the first peak wavelength may be changed to a wavelength more suitable for detected bacteria depending on, for example, the type of the detected bacteria. In this case, the illumination device 10 may include a plurality of light emitting devices whose first peak wavelengths have different wavelength regions, or may include a plurality of light emitting elements 3 having different peak wavelengths in one light emitting device as illustrated in Fig. 3.

For example, in the case where the illumination device 10 includes a bacteria detection sensor and an infrared sensor (human detecting sensor), when bacteria are detected and it is determined that there is no human, it is possible to increase the output of the light emitting device having the light with the first peak wavelength, and it is possible to efficiently perform sterilization without damaging a human. The sterilization effect can be provided by irradiation with light in a range of 360 to 430 nm.

The illumination device 10 may have a peak wavelength in a range of 315 to 400 nm. This can improve the sterilization effect. Alternatively, the illumination device 10 may have a peak wavelength in a range of 280 to 315 nm. This can further improve the sterilization effect on many bacteria, germs, and the like. Accordingly, when it can be determined that there is no human by an infrared sensor or the like, the illumination device 10 outputs an emission spectrum of UVA (315 to 400 nm) or UVB (280 to 315 nm) which is in the ultraviolet region, and can safely improve the strength of the sterilization effect.

The illumination device 10 according to the embodiment of the present disclosure can efficiently perform sterilization, presentation of light, and the like by adjusting the light of the light emitting device 1 with respect to what is detected by the sensor 8.

The light emitting device 1 according to the embodiment of the present disclosure and the illumination device 10 including the light emitting device 1 can reduce color variation of the light emitted from the light emitting device 1 caused by variation of the temperature of a plurality of phosphors 60 and variation of the fluorescence output at the peak wavelength emitted from each phosphor. That is, even when the output of the fluorescence at the peak wavelength emitted from one of the plurality of phosphors 60 varies, there is a high possibility that the color of the light from the light emitting device 1 can be maintained at a predetermined light color with the fluorescence emitted from the other phosphors. Thus, the light emitting device 1 according to the embodiment of the present disclosure can reduce color variation of the light emitted from the light emitting device 1 caused by variation of the emission intensity at the peak wavelength of the light emitted from the phosphor.

In the light emitting device 1 according to the embodiment of the present disclosure, light energy in a range of 360 to 430 nm accounts for 3% to 18% of light energy in a range of 360 to 780 nm. This makes it possible to obtain a sterilization effect by emitting light in the visible light region and also containing light in the near ultraviolet region. At this time, when the light in the near ultraviolet region (360 to 430 nm) accounts for 18% or less, it is possible to reduce deterioration or the like of the skin of the human body due to the light in the near ultraviolet region in daily life. When the light in the near ultraviolet region (360 to 430 nm) accounts for 3% or more, a sterilization effect can be exhibited.

In the light emitting device 1 according to the embodiment of the present disclosure, light energy in a range of 430 to 500 nm may account for 5% to 30% of light energy in a range of 360 to 780 nm. This makes it possible to obtain light having high color rendering properties while reducing the burden on the eyeballs or the like due to the intensity of blue color light (so-called blue light in a range of 430 to 500 nm).

In the light emitting device 1 according to the embodiment of the present disclosure, the half-width of a spectrum having a peak wavelength in a range of 360 to 430 nm may be 8 to 24 nm. This makes it possible to concentrate light energy in a specific region in the near ultraviolet region, and to effectively improve the sterilization effect on specific germs or the like.

In the light emitting device 1 according to the embodiment of the present disclosure, the light energy at 360 nm or less may be 2% or less of the light energy in a range of 360 to 780 nm. This can reduce a situation where light in the ultraviolet region becomes too strong. Thus, compared with the case where the light energy of 360 nm or less accounts for 2% or more, it is possible to reduce deterioration or the like of the skin of the human body due to light in the ultraviolet region.

In the light emitting device 1 according to the embodiment of the present disclosure, the half-width of a spectrum having a peak wavelength in a range of 430 to 500 nm may be 25 to 60 nm. This makes it possible to concentrate light energy in a specific region in the near ultraviolet region, and to effectively improve the sterilization effect on specific germs or the like.

In the light emitting device 1 according to the embodiment of the present disclosure, the emission intensity in a range of 360 to 400 nm may be 0.003 to 18 J/cm² per hour. This can reduce the possibility of skin deterioration or the like while achieving the sterilization effect.

In the light emitting device 1 according to the embodiment of the present disclosure, the irradiance of light in a range of 360 to 400 nm may be less than 10 W·m⁻². The above-described irradiance is the irradiance with which light is received from the light emitting device 1 at a distance at which a target organism or the like will approach the light emitting device 1 within the range in common life. This makes it possible to obtain a sterilization effect and satisfy the requirement of belonging to the exemption group of near-ultraviolet radiation damage to the eyes according to the JIS standard. Thus, it is possible to maintain the function as a light emitting device such as a room light and reduce the possibility of causing an injury to the eyeballs or the like.

In the light emitting device 1 according to the embodiment of the present disclosure, the irradiance of light in a range of 360 to 430 nm may be 33 W·m⁻² or less. This makes it possible to obtain a sterilization effect as presented in Fig. 10 and satisfy the requirement of belonging to the low risk group of near-ultraviolet radiation damage to the eyes according to the JIS standard. Thus, it is possible to maintain the function as a light emitting device such as a room light and reduce the possibility of causing an injury to the eyeballs or the like.

In the light emitting device 1 according to the embodiment of the present disclosure, the radiance of light in a range of 430 to 500 nm may be less than 100 W·sr⁻¹·m⁻². The above-described radiance is the irradiance with which light is received from the light emitting device 1 at a distance at which a target organism or the like will approach the light emitting device 1 within the range in common life. This makes it possible to obtain a sterilization effect and satisfy the requirement of belonging to the exemption group of retinal damage caused by blue light in accordance with the JIS standard, thereby reducing the possibility of causing an injury to the retina or the like.

The sterilization effect is determined by how much energy is given to certain germs. Thus, in a case where the light energy in the near ultraviolet region is large, the effect is exhibited even when the light application period is short, and in a case where the light energy in the near ultraviolet region is small, the effect is exhibited by increasing the light application period. However, when the light energy in the near ultraviolet region is too large, there is a concern that the light energy may lead to deterioration of the skin or the like, and when the light energy in the near ultraviolet region is too small, germs may increase before the effect is obtained. In contrast, with the above-described configuration, the light emitting device 1 according to the embodiment of the present disclosure can obtain a sterilization effect while using visible light used in daily life.

Furthermore, the light emitting device 1 according to the embodiment of the present disclosure can emit light having high color rendering properties approximate to the spectrum of sunlight. That is, the difference between the relative-intensity in the spectrum of sunlight and the relative-intensity in the emission spectrum of the light emitting device 1 according to the embodiment of the present disclosure can be reduced, and the light emitting device 1 that emits light approximate to sunlight can be produced.

Figs. 7 and 8 illustrate examples of fluorescence spectra including a plurality of phosphors 60 used in the light emitting device 1 of the present embodiment. Each spectrum is a spectrum represented by a relative-intensity when the highest emission intensity is 1. The emission spectra and the fluorescence spectra illustrated in Figs. 7 to 8 represent relative-intensities based on measured values.

The light emitting device 1 according to the embodiment of the present disclosure is used, for example, in a form in which a plurality of light emitting devices 1 is arranged in an illumination device used in an indoor place such as in a building, in a house, or the like. For example, with an illumination device for a living space, it is possible to construct an illumination environment as if irradiated with sunlight even in an indoor place. When used as an illumination device for appearance inspection of a coated object, for example, a passenger vehicle, an inspection environment as if irradiated with sunlight can be constructed even in an indoor place. When light close to sunlight is radiated even in an indoor place, it is possible to make a color appearance close to a color visible under sunlight (improvement in color rendering properties), and in a case where color inspection is performed, it is possible to perform the inspection more accurately in a state closer to a usage environment. In addition, the light emitting device 1 is useful for maintaining the health state of an organism because the light emitting device 1 can obtain a sterilization effect as well as a function of a general illumination device in activities in an indoor living environment, breeding of organisms for indoor breeding, and the like. Thus, the light emitting device 1 of the present embodiment is effective as an illumination device for facilities where sterilization is required such as a hospital or a spa, an animal breeding facility such as a pet shop, an indoor environment where germs easily increase such as a kitchen, a lavatory, or a bathroom. In addition, it is also effective in places where it is desired to make food look delicious and to suppress the increase in bacteria from a hygienic point of view, such as a refrigerator and a showcase of a Sushi restaurant.

Although the embodiment of the light emitting device 1 has been described, the illumination device 10 including a plurality of light emitting devices may have the same emission spectrum according to the embodiment. That is, similarly to the above-described light emitting device 1, by combining a plurality of light emitting devices including light emitting elements 3, the illumination device 10 has a first peak wavelength λ1 in a wavelength region of 360 to 430 nm and a plurality of peak wavelengths λx in a wavelength region of 360 to 780 nm.

Since the illumination device 10 has the first peak wavelength λ1 in the range of 360 to 430 nm, light in the near ultraviolet region closer to sunlight is radiated.

Since the emission spectrum of the light emitting device 1 or the illumination device 10 is close to the sunlight spectrum, the light emitting device 1 or the illumination device 10 provides comfort in daily life, has little influence on the eyes, skin, and the like of a human body, and can obtain a sterilization effect on various germs, molds, and the like.

Examples of the germs and molds for which sterilization with light in the near ultraviolet region is effective as described above include Escherichia coli, Staphylococcus aureus, drug-resistant Staphylococcus aureus, Salmonella, Shigella, Legionella, Bacillus cereus, Norovirus, Fusarium, Aspergillus niger, and Rhizopus.

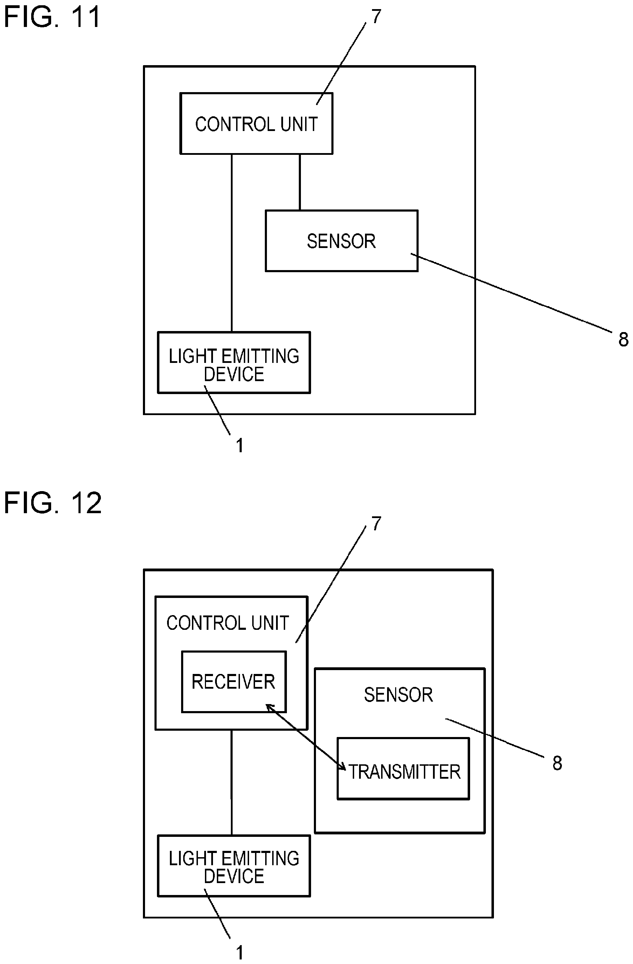

Fig. 12 is a result of actually verifying a sterilization effect by using the light emitting device 1 according to the embodiment of the present disclosure. The conditions of the light emitting device in this verification include that the irradiance in the near ultraviolet region is about 10 W·m⁻². From this result, it was possible to prove that even with little light in the ultraviolet region (less than 360 nm), light in the near ultraviolet region (360 to 430 nm) has a sterilization effect.

An example of an illumination device including the light emitting device 1 according to the present embodiment will be described below with reference to the accompanying drawings.

Fig. 9 is an external perspective view of an illumination device including a light emitting device according to the present embodiment. Fig. 10 is an exploded perspective view of the illumination device illustrated in Fig. 6. Fig. 11 is a perspective view illustrating a state in which the translucent substrate is removed from the housing of the illumination device illustrated in Fig. 9. The illumination device 10 includes a plurality of light emitting devices including light emitting elements. The light emitted from the plurality of light emitting devices has a first peak wavelength λ1 in a wavelength region of 360 to 430 nm and a plurality of peak wavelengths λx in a wavelength region of 360 to 780 nm.

The illumination device 10 includes a long housing 11 that is open upward, a plurality of light emitting devices 1 arranged in a line in the housing 11 in the longitudinal direction, a long wiring board 12 on which the plurality of light emitting devices 1 is mounted, and a long translucent substrate 13 that is supported by the housing 11 and that closes the opening of the housing 11.

While the spectrum of the light emitting device 1 has been described, the spectrum of the illumination device 10 may be as described above. In this case, the light emitting element 3 also has a first peak wavelength λ1 in a range of 280 to 315 nm, and can be reproduced by appropriately changing the types of phosphors.

The housing 11 has a function of holding the translucent substrate 13 and a function of dissipating heat generated by the light emitting devices 1 to the outside. The housing 11 is made of, for example, metal such as aluminum, copper, or stainless steel; plastic; resin; or the like. The housing 11 stands erect from a bottom portion 21a extending in the longitudinal direction and both end portions of the bottom portion 21a in the width direction. The housing 11 further includes a main body portion 21 having a pair of long support portions 21b extending in the longitudinal direction and being open upward and on both sides in the longitudinal direction, and two lid portions 22 that respectively close openings on one side and the other side in the longitudinal direction of the main body portion 21. A holding portion in which recesses for holding the translucent substrate 13 are formed to face each other in the longitudinal direction is provided in an upper portion of each support portion 21b inside the housing 11. The length of the housing 11 in the longitudinal direction is set to, for example, 100 mm or more and 2000 mm or less.

The wiring board 12 is secured to the bottom surface of the housing 11. As the wiring board 12, for example, a printed substrate such as a rigid substrate, a flexible substrate, or a rigid flexible substrate is used. The wiring pattern of the wiring board 12 and the wiring pattern of the substrate 2 in the light emitting device 1 are electrically connected to each other with solder or a conductive adhesive therebetween. Then, a signal from the wiring board 12 is transmitted to the light emitting element 3 through the substrate 2, and the light emitting element 3 emits light. Power is supplied to the wiring board 12 from an external power source via wiring.

The translucent substrate 13 is made of a material through which the light emitted from the light emitting device 1 is transmitted, and is made of, for example, a light-transmitting material such as an acrylic resin or glass. The translucent substrate 13 is a rectangular plate body and has a length in the longitudinal direction set to, for example, 98 mm or more and 1998 mm or less. The translucent substrate 13 is inserted into the recess formed in each of the support portions 21b described above from the opening on one side or the other side in the longitudinal direction of the main body portion 21. Then, by sliding in the longitudinal direction, the translucent substrate 13 is supported at positions separated from the plurality of light emitting devices 1, by the pair of support portions 21b. The illumination device 10 is configured by closing the openings on the one side and the other side in the longitudinal direction of the main body portion 21 with the lid portions 22.

Although the above-described illumination device 10 is a linear light emitting illumination device in which the plurality of light emitting devices 1 is linearly arranged, the illumination device 10 is not limited thereto. The illumination device may be a surface emitting illumination device in which the plurality of light emitting devices 1 is arranged in a matrix form or a staggered grid form.

As described above, the light emitting device 1 according to the embodiment of the present disclosure includes, as the phosphors included in one wavelength conversion member 6, the five types of phosphors including the phosphor that emits blue fluorescence, the phosphor that emits blue-green fluorescence, the phosphor that emits green fluorescence, the phosphor that emits red fluorescence, and the phosphor that emits fluorescence in the near infrared region. However, the configuration is not limited thereto, and two types of wavelength conversion members may be provided. When two types of wavelength conversion members are provided, different phosphors may be dispersed or phosphors may be dispersed in different combinations in a first wavelength conversion member and a second wavelength conversion member. These two wavelength conversion members may be provided in one light emitting device, and light beams emitted through the respective wavelength conversion members may be mixed. In this way, the color rendering properties of the emitted light can be easily controlled.

The emission spectrum of the produced light emitting device 1 is the emission spectrum illustrated in Fig. 7.

The present disclosure is not limited to the examples of the embodiments described above, and various modifications for such as numerical values are possible. Various combinations of features in the present embodiment are not limited to the examples of the embodiment described above.

### Reference Signs List

- 1: light emitting device
- 1a: first light emitting device
- 1b: second light emitting device
- 10: illumination device
- 11: housing
- 12: wiring board
- 13: translucent substrate
- 2: substrate
- 21: main body portion
- 21a: bottom portion
- 21b: support portion
- 22: lid portion
- 3: light emitting element
- 4: frame body
- 5: sealing member
- 6: wavelength conversion member
- 60: phosphor
- 7: control unit
- 71: first selection unit
- 72: second selection unit
- 8: sensor
- 81: sensor unit
- λ1: first peak wavelength
- λ2: second peak wavelength

## Claims

1. An illumination device comprising:
a light emitting device that emits light having a first peak wavelength in a wavelength range of 360 to 430 nm and a second peak wavelength in a wavelength range of 430 to 700 nm;
a control unit that controls an emission spectrum of the light emitted from the light emitting device; and
a sensor that includes a sensor unit and that transmits a signal received by the sensor unit to the control unit,
wherein the control unit controls the emission spectrum based on a signal detected by the sensor.

2. An illumination device comprising:
a plurality of light emitting devices that emits light having a first peak wavelength in a range of 360 to 430 nm and a second peak wavelength in a range of 430 to 700 nm;
a control unit that controls an emission spectrum of light obtained by combining the light emitted from each of the plurality of light emitting devices; and
a sensor that includes a sensor unit and that transmits a signal received by the sensor unit to the control unit,
wherein the control unit controls the emission spectrum based on a signal detected by the sensor.

3. An illumination device comprising:
a first light emitting device that emits light having a first peak wavelength in a range of 360 nm to 430 nm;
a second light emitting device that emits light having a second peak wavelength in a range of 430 nm to 700 nm;
a control unit that controls an emission spectrum obtained by combining a first emission spectrum of the light emitted from the first light emitting device and a second emission spectrum of the light emitted from the second light emitting device; and
a sensor that includes a sensor unit and that transmits a signal received by the sensor unit to the control unit,
wherein the control unit controls the emission spectrum based on a signal detected by the sensor.

4. The illumination device according to any one of claims 1 to 3, wherein the sensor transmits the detected signal to the control unit through wireless communication.

5. The illumination device according to any one of claims 1 to 4, wherein the sensor is an optical sensor.

6. The illumination device according to claim 5, wherein the optical sensor is an infrared sensor.

7. The illumination device according to any one of claims 1 to 4, wherein the sensor is a particle sensor.

8. The illumination device according to any one of claims 1 to 4, wherein the sensor is an odor sensor.

9. The illumination device according to any one of claims 1 to 4, wherein the sensor is a bacteria detection sensor.

10. The illumination device according to any one of claims 1 to 9, wherein the control unit controls a voltage and/or a current of the light emitting device.

11. The illumination device according to any one of claims 1, and 4 to 10,
wherein the light emitting device includes a plurality of light emitting elements, and
wherein the control unit selects a light emitting element to emit light from the plurality of light emitting elements.

12. The illumination device according to any one of claims 2, and 4 to 11, wherein the control unit selects a light emitting device to emit light from the plurality of light emitting devices.

13. The illumination device according to any one of claims 3, and 4 to 10, wherein the control unit selects a light emitting device to emit light from the first light emitting device and/or the second light emitting device.

14. The illumination device according to any one of claims 1, and 4 to 10,
wherein the light emitting device includes a plurality of light emitting elements, and
wherein the control unit adjusts an emission intensity of at least one light emitting element among the plurality of light emitting elements.

15. The illumination device according to any one of claims 1 to 14, wherein the emission spectrum has a peak wavelength in a range of 315 to 400 nm.

16. The illumination device according to any one of claims 1 to 15, wherein the emission spectrum has a peak wavelength in a range of 280 to 315 nm.

17. The illumination device according to any one of claims 5 to 9,
wherein the sensor includes a plurality of sensors, and
wherein the control unit controls the emission spectrum based on a signal detected by each of the sensors.
